# EUROPEAN PATENT APPLICATION

(11) **EP 2 179 707 A1**
(43) Date of publication of application: **28.04.2010**
(21) Application number: 08425683.3
(22) Date of filing: 21.10.2008
(51) Int. Cl.: A61F 2/00, A61B 17/04

(54) **Device for the treatment of female urinary incontinence and genital prolapse**

(71) Applicant: BIOENGINEERING LABORATORIES S.p.A., 22063 Cantù (CO) (IT)
(72) Inventor: Salvatore, Stefano, 21000 Varese (IT); Athanasiou, Stavros, 11528 Athens (GR); Cardozo, Linda, Potter St. Hill Pinner Mddx HA5 3XH (GB); Greco, Francesco, 22063 Cantù (CO) (IT); Bongini, Paolo, 22063 Cantù (CO) (IT)
(74) Representative: Ferraiolo, Rossana

(57) **Abstract**

The device comprises anchoring means (3) in the form of an arrowhead or wedge applied at the free extremities of a tape (2) made of a synthetic macro-porous material or a prosthetic material replacing the facial tissue, where said anchoring means are fitted with gripping elements provided along the profile and on the surface in the form of projecting crests (4, 5, 7, 8) and pins (9) and a concavity (6) provided on each of the opposite faces to position the extremities of a forceps or other suitable instrument, while said cavities (6) are fitted with transversal gripping elements (13) and the anchoring means are optionally curved in their main plane.

## Description

### Basis of the invention

The present invention refers to a device for treating female urinary stress incontinence, and in particular to a tape-like device (hereinafter known as a mini-sling) fitted with anchoring means for emplacing and anchoring the device in the pertinent anatomical position.

The invention also refers to a device for treating female genital prolapse, and in particular to anchoring means for emplacing and anchoring a prosthesis device in the pertinent anatomical position.

Urinary incontinence is a disturbance prevalently affecting the female sex, and doubtlessly occasioning hygienic, economical and social problems.

The female urinary incontinence can be classified into the following three types:
- urge incontinence where urine loss is accompanied or immediately preceded by an impelling urge to pass urine, so as to be non-controllable or difficult to control;
- Stress-induced incontinence, consisting of an involuntary urine loss on effort, coughing or sneezing;
- Mixed urinary incontinence, consisting of an involuntary urine loss associated both with a sudden urge to pass urine as well as with effort, coughing or sneezing.

Specific treatments are known for each of these types of incontinence, comprising physiotherapy, pharmacological treatment and the use of surgical devices.

Genital prolapse is also one of the major causes in the gynecological sector, and may involve the frontal, upper or rear regions separately or jointly. A surgical treatment may involve the usage of native tissue or prostheses for strengthening or replacing the compromised tissues. The prostheses may be positioned and fastened, by sutures or specific needle passes, to muscular-fascial or ligament structures (for instance, through the transobturatory or transgluteal passage in case a traditional fascial surgery is not desired, thus resorting to specific surgical kits).

The present invention thus falls, as already mentioned, within the field of surgical devices for treating female urinary stress incontinence and genital prolapse, which are in the majority of cases calling for a surgical approach and have in recent years been the object of important research and development aimed at surgical devices of low invasiveness and at their relative surgical techniques.

### State of the Art

There are known surgical devices such as the so-called "mini-slings" applied with very recent minimally invasive techniques and positioned in the muscular-fascial obturatory complex in the case of a suburethral mini-sling for treating female urinary stress incontinence, and in the muscular-fascial-ligamental pelvical structures in the case of treating female genital prolapse.

The documents US 7,297,102 Smith et al., US 2002/0099260 Suslan et al and US 2007/0043255 O'Donnell illustrate devices of a "mini-sling" type, emplacing below the urethra.

The first document shows a mini-sling fitted at its two opposite extremities with extensions for attaching to the surrounding tissue through indentations provided along the outer profile of these extensions, and being coplanar with the same.

The second document illustrates a mini-sling whose pointed extensions do not present elements for attaching to the surrounding tissue.

The third document illustrates a mini-sling fitted with extensions of a triangular shape, for attaching to the tissue through external flexures made of a synthetic or biodegradable material.

Other known devices are those commercialized by Ethicon and AMS under the commercial names of "TVT Secure" and "MiniArc", respectively. The first presents extensions that are essentially flat, while the second presents extensions fitted with a small self-attaching tip.

### Disadvantages of the State of the Art

The conventional devices used in connection with traditional surgical techniques involve rather invasive operations that impose prolonged hospitalization and whose success may be compromised by post-operative complications. The most recent devices such as the "mini-slings" for treating urinary stress incontinence present limitations and drawbacks; the correct positioning of such devices may turn out to be difficult, and because of the reduced possibility of adjusting the position and tension of the "mini-sling" in an executive phase, drawbacks may occur that compromise the operation's favorable outcome.

In general, despite the fact that the devices demand a local anesthesia and an average hospitalization of 1 - 2 days, complications may occur such as requiring a further and more invasive surgical intervention, with consequently lengthened hospitalization periods.

Even for treating genital prolapse, the surgical techniques of pelvic reconstruction call for a specific competence acquired by extensive surgical training. On the other hand, the diffusion of innovative surgical devices, of new surgical kits and the lack of standardization may offer disadvantages and drawbacks related to the fact that such novel devices are passed through anatomical areas that may damage organs or their associated vessels.

### Exposition of the invention

The technical problem the invention plans to address is to provide a device capable of being attached and adjusted in its pertinent anatomical position so as to eliminate the drawbacks of the devices of the known art.

Another scope of the present invention is to furnish a device for treating female genital prolapse, in particular for positioning prostheses in the pelvic hollow.

The device for treating stress-induced female urinary incontinence is constituted of a tape such as employed in the conventional "mini-slings" and of anchoring means of any type capable of being inserted in a surrounding tissue and preferably of an arrow-shaped or wedge-shaped type, applied at the extremities of the tape and **characterized in that** the anchoring means comprise:
- a plurality of attaching elements provided along their profile and on their surfaces, in the form of projecting peaks and pins to take hold in the surrounding tissue;
- a cavity formed in each of the opposite faces, to position the extremities of a forceps or other suitable instrument in the operating phase;
- a plurality of transversal elements in the mentioned opposite concavities, to facilitate the grip taken by the extremities of a forceps or other suitable instrument in the operating phase.

The anchoring means are advantageously curved in their principal plane, according to a curving radius capable of easing their insertion during the executive phase.

The interior of the anchoring means optionally comprises a central core of a more rigid material such as for instance polypropylene, which is directly applied to the respective opposite extremities of the tape.

The anchoring means are made of materials chosen from a range of known re-absorbable materials in the surgical field, such as poly-glycolic acid, Poliglactin 910 (a glycolic acid/lactic acid copolymer), poly-dioxanone, poly-glyconate and other known materials capable of guaranteeing an adequate reaction time in the guest tissue.

Each anchoring means is applied at one of the opposite extremities of the tape, according to fastening ways that depend on the type of material they're made of, and comprise an overprinting of a type particularly recommended in the case of anchoring means made of poly-propylene, a gluing with an appropriate resin or a welding based on radio-frequency or ultrasounds.

The anchoring means of the present invention have a thickness in the range of 0.8 to 4.5 mm and a bottom length at the tape connecting level in the range of 10.5 to 14.0 mm.

The gripping elements provided along the profile and on the surface of the anchoring means in the form of peaks and pins have a length in the range of 0.2 to 5.0 mm.

The curving radius of the anchoring means is in the range of 40 to 140 mm for the inner curving, and of 40 to 60 mm for the outer curving.

The anchoring means of the present invention may be applied to a prosthesis device of a biological, synthetic or mixed type for treating female genital prolapse.

In this case, the anchoring means have been fitted at the extremities of the prosthetic material replacing the fascial tissues at the points corresponding to lugs previously applied to such extremities.

### Advantages of the invention

The anchoring means provided with opposite concavities, optionally fitted with transversal gripping elements, allow an easy and safe gripping of the invented device by a forceps or other suitable surgical instrument.

The resulting grip is such as to allow the essentially arrow-shaped anchoring means to act as an extension of the forceps itself, so as to form a single rigid penetrating unit.

This facilitates a gradual step-wise insertion of a first extremity and then of the other extremity in an operating phase.

The gripping elements provided along the profile and on the surface of the anchoring means according to the present invention facilitate the positioning and the possible adjusting of the device in its ideal position.

Even in the case of a pelvic prolapse, the anchoring means applied to the prosthetic material allow a facilitated insertion and a precise positioning of the prosthesis.

The use of a re-absorbable material for the production of the anchoring means provides a result that leaves only a minimal part of foreign material inside the tissue for a certain time following the operation. After re-absorbing, the device's anchoring ability is such as to guarantee an effective and durable retention.

### Examples of embodiment

The invention will be illustrated in detail with reference to some examples of embodiment and the attached drawings, where:
Figure 1 is a perspective view of a first embodiment of the device;
Figure 2 is a top plan view of a second embodiment;
Figure 3 is a top plan view and a side view of a third embodiment;
Figures 4 and 5 are side sectional views of a fourth embodiment; and
Figure 6 is a top plan view of the fourth embodiment.

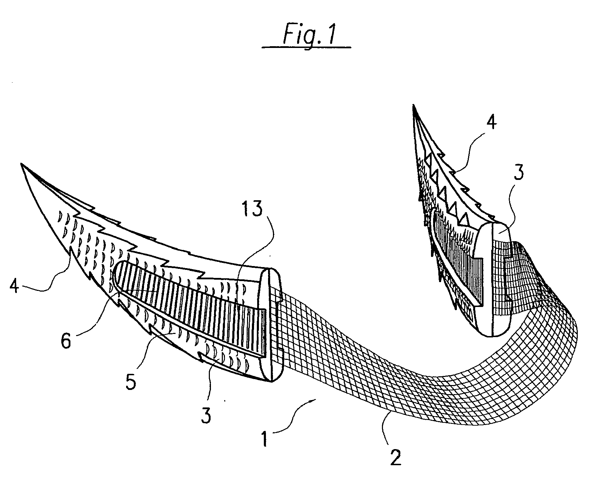

Fig. 1 shows a device 1 comprising a tape 2 of a type used for conventional mini-slings, of suitable elasticity, length and strength. It is made of a synthetic material of a macro-porous and mono-filamentous poly-propylene type, but may also employ Dacron, polyethylene or other materials suitable for the purpose.

The figure shows that the anchoring means 3 having an essentially arrow-shaped form are applied at the free extremities of the tape. They are made of a re-absorbable material known in the surgical field, such as polyglycolic acid, and are attached to the free extremities of the tape by overprinting.

The figure shows a plurality of gripping elements 4, 5 provided along the profile and on the surface of each anchoring means 3, and a concavity 6 carrying a plurality of transversal elements 13 serving, together with equal concavities and elements provided on the opposite face, to facilitate the gripping of the opposite extremities of a forceps.

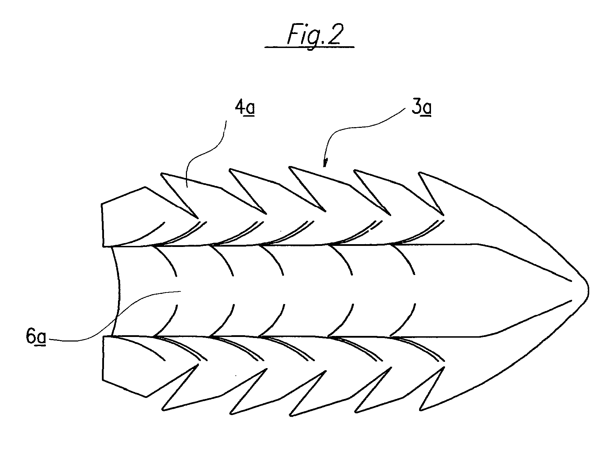

Fig. 2 shows an alternative embodiment of an anchoring means 3 a shaped as an arrow head, carrying gripping elements 4a on its opposite lateral profiles and on two opposite concavities 6a, which are likewise provided to facilitate the gripping of the opposite extremities of a forceps.

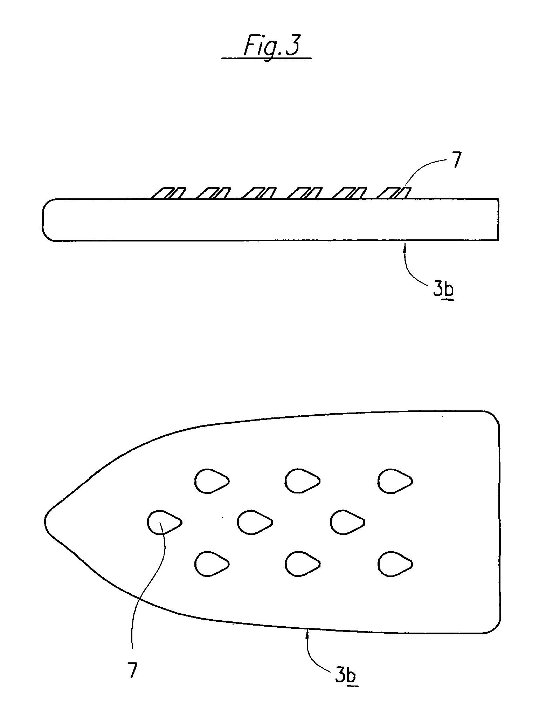

Fig. 3 shows a second alternative embodiment of an anchoring means 3b carrying on each of its opposite surfaces a plurality of gripping elements 7 of a truncated conical shape, which are tilted toward the inserting direction of the tape (not shown).

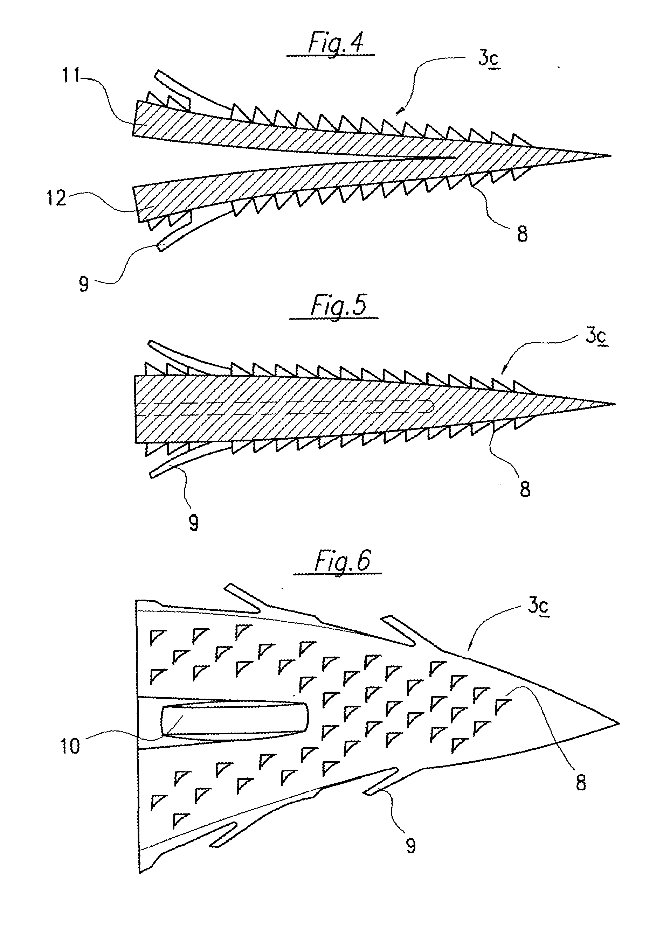

Figs. 4 and 5 illustrate side sectional views of a variant 3c of the anchoring means of Fig. 1 carrying gripping means of a wedge-shaped form (8), which are uniformly distributed over the surface, and elements in the form of pins (9) distributed along the profile.

Fig. 4 shows half-portions 11, 12 of the anchoring means acting together to form the body of the anchoring means. Once applied to the free extremities of the tape (not shown) and attached to the latter by overprinting, they form the body of the anchoring means 3c shown in Fig. 5.

The mentioned half-portions 11, 12 are advantageously identified with respect to each other by a different color (not shown) or other type of identifying symbols, so as to render the respective sides of the device immediately recognizable by an operator.

Fig. 6 is a top plan view of the variant 3c of the anchoring means of Fig. 4, showing one of the opposite grooves 10, which are provided to facilitate the gripping of the opposite extremities by a forceps or other suitable surgical instrument.

The method of inserting the invented device substantially comprises the phases of:
a) picking up the device with a forceps or other suitable surgical device at a point corresponding to the opposite concavities 6 (Figs. 1, 2) or to the insert 10 (Fig. 6) of one of the two anchoring means applied to one of the free extremities of the tape;
b) inserting the device by positioning said anchoring means in a first position from which the device can be moved forward according to the following point e);
c) picking up the device at a point corresponding to the opposite concavities of the other anchoring means applied to the other free extremity of the tape;
d) positioning said other anchoring means in a second position from which the device can be moved forward according to the following point e);
e) gradually advancing the device by stepwise positioning first one and then the other anchoring means, while symmetrically acting on each extremity;
f) adjusting the position of the anchoring means, while acting on one or both of them to obtain the correct positioning of the tape;
g) releasing the device in the ideal position, once the opposite extremities of the forceps have been released.

The method of using the invented device for treating genital prolapse comprises the following phases:
i) applying the anchoring means of the present invention to the prosthetic device, at a point corresponding to lugs previously mounted on the respective extremities of the prosthesis;
ii) gradually inserting the device by stepwise positioning of each anchoring means while acting symmetrically on each extremity to achieve the correct positioning of the device;
iii) releasing the device in the ideal position, once the opposite extremities of the forceps have been released.

## Claims

1. Device for treating female urinary stress incontinence, wherein a tape (2) of a synthetic macro-porous material carries at its free extremities anchoring means (3) of a substantially arrow-head or wedge shaped type, **characterized in that** they comprise:
- a plurality of gripping elements provided along their profile and on their surface in the form of projecting crests (4, 5, 7, 8) and pins (9) to take hold in the surrounding tissue;
- a concavity (6) provided on each of the opposite faces, to position the extremities of a forceps or other suitable surgical instrument.

2. Device according to claim 1, **characterized in that** the concavities (6) are fitted with a plurality of transversal gripping elements (13).

3. Device according to the claims 1, 2 and 3, **characterized in that** the anchoring means (3) are curved in their main plane according to a curving radius in the range of 40 to 140 mm for the internal curving, and of 40 to 60 mm for the external curving.

4. Device according to claim 1 and 2, **characterized in that** the anchoring means (3) have a thickness in the range of 0.8 to 4.5 mm and a bottom length in the range of 10.5 to 14.0 mm at the point of connecting to the tape.

5. Device according to claim 1 and 2, **characterized in that** the gripping elements (4, 5, 7, 8, 9) have a length in the range from 0.2 to 0 5.0 mm.

6. Device according to the claims 1 to 5, **characterized in that** the interior of the anchoring means comprises a central core of greater rigidity that the outer portion.

7. Device according to the claims 1 to 6, **characterized in that** the anchoring means are made of a re-absorbable material.

8. Device according to the claims 1 to 7, **characterized by** the fact that the half-portions of the anchoring means (11, 12) are identified with respect to each other by a different color or other type of identifying symbols.

9. Device for treating female genital prolapse, **characterized in that** the anchoring means according to the claims 1 to 8 are applied at the extremities of a prosthetic material replacing the fascial tissues at a point corresponding to lugs previously applied to said extremities.

## Amended claims

### Amended claims in accordance with Rule 137(2) EPC.

**1.** Device for treating female urinary stress incontinence, wherein the ends of a tape (2) of a synthetic macro-porous material carry anchoring means (3) of a substantially arrow-head or wedge shaped type, a plurality of gripping elements are provided along the profile and on the surface of the anchoring means in the form of projecting crests (4, 5, 7, 8) and **filiform appendixes** (9) to take hold in the surrounding tissue, **characterized in that** the anchoring means (3) are curved in their main plane according to a radius of curvature in the range of 40 to 140 mm for the internal curve and of 40 to 60 mm for the external curve.

**2.** Device according to claim 1 **characterized in that** a concavity (6) is provided on each of the opposite faces in the anchoring means to position the clamping extremities of a forceps or other suitable surgical instrument.

**3.** Device according to claims 1, 2 **characterized in that** the concavities (6) are fitted with a plurality of transversal gripping elements (13).

**4.** Device according to the claims 1 to 3 **characterized in that** the anchoring means (3) have a thickness in the range of 0.8 to 4.5 mm and a bottom length in the range of 10.5 to 14.0 mm at the point of connecting to the tape.

**5.** Device according to the claims 1 to 3 **characterized in that** the **projecting crests** (4, 5, 7, 8) and **filiform appendixes** (9) have a length in the range from 0.2 to 5.0 mm.

**6.** Device according to the claims 1 to 5 **characterized in that** the interior of the anchoring means comprises a central core (3d) of **rigidity greater than that of the outer portion** (3e).

**7.** Device according to the claims 1 to 6, **characterized in that** the outer portion (3e), the central core (3d), the projecting crests (4, 5, 7, 8) and the filiform appendixes (9) of the anchoring means are made of re-absorbable material.

**8.** Device according to the claims 1 to 7, **characterized in that** the anchoring means (3) are identified with respect to each other by a different color or other type of identifying marks for showing an operator the proper side of introduction.

**9.** Device for treating female genital prolapse, **characterized in that** the anchoring means according to the claims 1 to 8 are applied at the ends of a prosthetic material which replaces the fascial tissues at a point corresponding to lugs previously applied to said ends.
